# EUROPEAN PATENT APPLICATION

(11) **EP 3 103 392 A1**
(43) Date of publication of application: **14.12.2016**
(21) Application number: 16172102.2
(22) Date of filing: 31.05.2016
(51) Int. Cl.: A61B 5/1455, A61B 5/024, A61B 5/00

(54) **MEDICAL APPARATUS, PHYSIOLOGICAL PARAMETER ANALYZING METHOD AND PHYSIOLOGICAL PARAMETER ANALYZING PROGRAM**

(30) Priority: 09.06.2015 JP 2015116888
(71) Applicant: Nihon Kohden Corporation, Shinjuku-ku Tokyo (JP)
(72) Inventor: UKAWA, Teiji, Shinjuku-ku, Tokyo (JP)
(74) Representative: Grünecker Patent- und Rechtsanwälte PartG mbB

(57) **Abstract**

A medical apparatus includes a light emitter configured to emit light beams of different wavelengths to living tissue, a light detector configured to detect the light beams of different wavelengths emitted from the light emitter, an acquiring section that calculates absorbances of arterial blood for the respective light beams of different wavelengths based on the light beams detected by the light detector, that acquires a pulse waveform based on the absorbances, and that acquires a parameter corresponding to an oxygen saturation from a ratio of the absorbances, a detecting section that detects variation of the parameter corresponding to the oxygen saturation acquired by the acquiring section, and an evaluating section that evaluates the pulse waveform acquired for a purpose of pulse wave analysis by the acquiring section based on a result of the detection by the detecting section.

## Description

### BACKGROUND

The present invention relates to a medical apparatus, a method for analyzing a physiological parameter, and a program for analyzing a physiological parameter.

A pulse oximeter is used as an apparatus which may measure in real time the arterial oxygen saturation and the arterial pulse wave without collecting blood. A pulse oximeter is attached to the fingertip or the like of the subject, illuminates living tissue including arterial blood with light beams of different wavelengths, and measures the oxygen saturation SpO2 of arterial blood, and the pulse wave (photoelectric capacity pulse wave) based on transmitted or reflected light beams which are received through the living tissue.

The journal, Ukawa Teiji, "Hemodynamic Monitoring and Pulse Oximeter", Japanese journal of intensive care medicine, 2014, vol. 38(3), 167-175, discloses an attempt to analyze the waveform of a pulse wave (hereinafter, such a waveform is often referred to merely as "pulse waveform") detected by a pulse oximeter, thereby acquiring physiological parameters such as the pulse wave amplitude, and pulse interval which are important information in monitoring of respiratory circulation.

However, noise due to a body motion such as motion of the hand or finger of the subject, external light which enters from the outside, or the like may be often mixed in a pulse wave detected by a pulse oximeter. When such an artifact is mixed in a detected pulse wave, the resulting pulse waveform is not the original waveform which correctly indicates the condition of the subject. Also in physiological parameters such as the pulse interval, and pulse wave amplitude which are obtained by analyzing the pulse waveform, therefore, the accuracy and the correctness are lowered. In the case where treatment is performed based on physiological parameters which are measured in a state where a pulse wave is mixed with noise and the pulse waveform is disturbed, and which is therefore low in accuracy and correctness, there is a possibility that an adequate determination of condition of the subject for treatment cannot be performed.

The present invention has been conducted in view of the above circumstances. It is an object of the present invention to provide a medical apparatus, and method and program for analyzing a physiological parameter which may evaluate the waveform of a pulse wave with consideration of noise mixture in the pulse wave.

### SUMMARY

(1) According to an aspect of the present invention, a medical apparatus includes a light emitter that is configured to emit light beams of different wavelengths to living tissue, a light detector that is configured to detect the light beams of different wavelengths emitted from the light emitter, through the living tissue, an acquiring section that calculates absorbances of arterial blood for the respective light beams of different wavelengths, based on the light beams detected by the light detector, that acquires a pulse waveform based on the absorbances, and that acquires a parameter corresponding to an oxygen saturation from a ratio of the absorbances, a detecting section that detects variation of the parameter corresponding to the oxygen saturation acquired by the acquiring section, and an evaluating section that evaluates the pulse waveform that is acquired for a purpose of pulse wave analysis by the acquiring section, based on a result of the detection by the detecting section.
(2) According to another aspect of the present invention, a method for analyzing a physiological parameter, the method being used in a medical apparatus having a light emitter that is configured to emit light beams of different wavelengths to living tissue including arterial blood, and a light detector that is configured to detect the light beams of different wavelengths emitted from the light emitter, through the living tissue, includes calculating absorbances of the arterial blood for the respective light beams of different wavelengths, based on the light beams detected by the light detector, acquiring a pulse waveform based on the absorbances and a parameter corresponding to an oxygen saturation from a ratio of the absorbances, detecting variation of the parameter corresponding to the oxygen saturation acquired in the acquiring, and evaluating the pulse waveform which is acquired for a purpose of pulse wave analysis in the acquiring, based on a result of the detection in the detecting.
(3) According to another aspect of the present invention, a program executed in an apparatus for processing a physiological parameter obtained from a light emitter that is configured to emit light beams of different wavelengths to living tissue, and a light detector that is configured to detect the light beams of different wavelengths emitted from the light emitter, through the living tissue, causes the apparatus to execute calculating absorbances of the arterial blood for the respective light beams of different wavelengths, based on the light beams detected by the light detector, acquiring a pulse waveform based on the absorbances and a parameter corresponding to an oxygen saturation from a ratio of the absorbances, detecting variation of the parameter corresponding to the oxygen saturation acquired in the acquiring, and evaluating the pulse waveform which is acquired for a purpose of pulse wave analysis in the acquiring, based on a result of the detection in the detecting.

According to the medical apparatus of the present invention, variation of the oxygen saturation is detected, and a pulse waveform which is acquired for the purpose of pulse wave analysis is evaluated based on the detected variation of the oxygen saturation. Therefore, a physiological parameter is output with consideration of effects of noise. Consequently, the user may adequately determine the condition of the subject based on the output physiological parameter.

### BRIEF DESCRIPTION OF DRAWINGS

Fig. 1 is a view diagrammatically illustrating the configuration of a medical apparatus of an embodiment of the present invention.
Fig. 2 is a block diagram illustrating the function of the medical apparatus illustrated in Fig. 1.
Figs. 3A to 3C illustrate examples of results of measurements of the pulse waveform and the oxygen saturation.
Fig. 4 is a flowchart illustrating the procedure of a physiological parameter analyzing process executed in the medical apparatus.
Fig. 5 illustrates an example of table information indicating relationships between the reliability of the pulse waveform and the reliabilities of physiological parameters.
Fig. 6 illustrates a process of selecting a physiological parameter(s) which is to be output, based on the reliability of the physiological parameter.

### DETAILED DESCRIPTION OF EMBODIMENTS

Hereinafter, embodiments of the present invention will be described with reference to the accompanying drawings. In the description of the drawings, the identical components are denoted by the same reference numerals, and duplicated description is omitted. In the drawings, the dimension ratios may be sometimes different from the actual ratios.

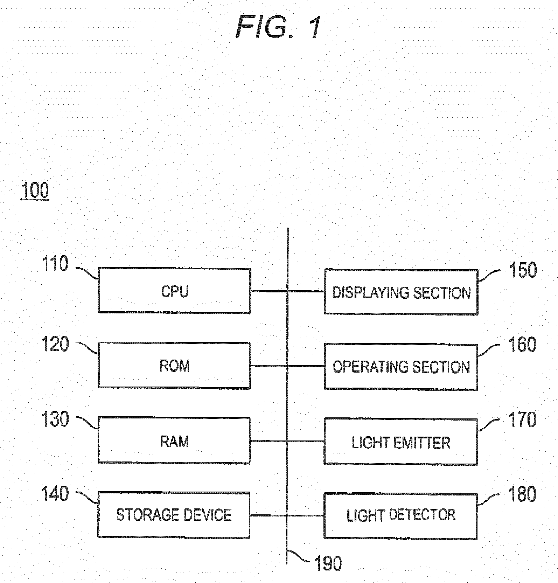

Fig. 1 is a view diagrammatically illustrating the configuration of a medical apparatus of an embodiment of the present invention.

The medical apparatus 100 of the embodiment is a pulse oximeter which is to be attached to the finger tip or the like of the subject to measure the arterial pulse wave and the arterial oxygen saturation.

As illustrated in Fig. 1, the medical apparatus 100 may include a CPU 110, a ROM 120, a RAM 130, a storage device 140, a displaying section 150, an operating section 160, a light emitter 170, and a light detector 180. These components are connected to one another through a bus 190 for exchanging signals.

The CPU 110 controls the above-described components and performs various calculation processes in accordance with programs which are recorded in the ROM 120 and the storage device 140. The CPU 110 executes the programs to function as an acquiring section, a detecting section, an evaluating section, an analyzing section, a determining section, and an outputting section. The ROM 120 stores various programs and data. The RAM 130 functions as a working area to temporarily store programs and data.

The storage device 140 stores various programs including an operating system, and various data. Information for evaluating the reliability of a a pulse waveform based on variation of the oxygen saturation, that for evaluating the reliabilities of various physiological parameters based on the reliability of the pulse waveform, and the like are stored in the storage device 140.

The displaying section 150 is, for example, a liquid crystal display, and displays various kinds of information such as a measured pulse waveform and the oxygen saturation.

The operating section 160 is used for performing various inputs. The operating section 160 may include operation keys which are realized by software with a touch panel in the displaying section 150, operation buttons which are disposed as hardware, and the like.

The light emitter 170 illuminates living tissue including arterial blood with light beams of different wavelengths. Examples of the light beams of different wavelengths are light beams of two wavelengths, i.e., a red light beam of a wavelength of 660 nm and an infrared light beam of a a wavelength of 940 nm. For example, the light emitter 170 is configured by two light emitting diodes. The light emitter 170 emits the light beams of two wavelengths toward the arterial blood flow in the fingertip or the like of the subject.

The light detector 180 receives the light beams of two wavelengths which are emitted from the light emitter 170 to the living tissue, through the living tissue. The light detector 180 may receive transmitted light beams which are transmitted through the living tissue, or reflected light beams which are reflected from the living tissue.

The medical apparatus 100 may include components other than the above-described components, or may not include a part of the above-described components.

Next, the function of the medical apparatus 100 will be described.

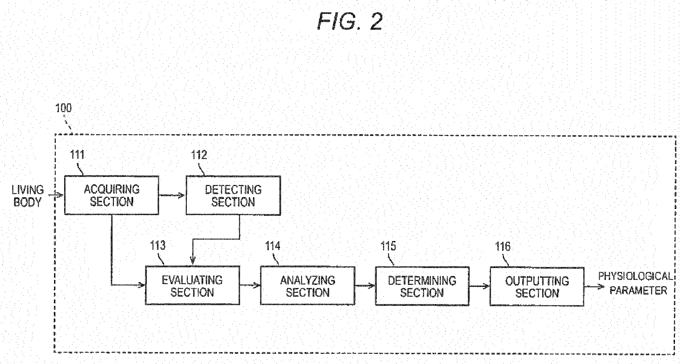

Fig. 2 is a block diagram illustrating the function of the medical apparatus illustrated in Fig. 1.

As illustrated in Fig. 2, the medical apparatus 100 may include an acquiring section 111, a detecting section 112, an evaluating section 113, an analyzing section 114, a determining section 115, and an outputting section 116.

The acquiring section 111 calculates absorbances of the arterial blood, from the light beams of two wavelengths detected by the light detector 180, and acquires the arterial pulse waveform based on the calculated absorbances. The pulse waveforms acquired from the light beams of two wavelengths are similar to each other. Therefore, the acquiring section 111 may employ one of the pulse waveforms as the arterial pulse waveform. The acquiring section 111 further acquires the arterial oxygen saturation from a ratio of absorbances for the light beams of two wavelengths. The acquiring section 111 may acquire a ratio of absorbances based on absorbances at an arbitrary timing. For example, the acquiring section 111 may acquire a ratio of absorbances at a timing when the absorbances are maximum for each pulse, or that at a specific timing.

The detecting section 112 detects variation of the parameter corresponding to the oxygen saturation acquired by the acquiring section 111. Although, here, the oxygen saturation itself is described as the parameter corresponding to the arterial oxygen saturation, the parameter corresponding to the oxygen saturation may be the absorbance ratio itself, or a function of the absorbance ratio which corresponds one-to-one to the oxygen saturation. The function of the absorbance ratio is the square, square root, or linear function of the absorbance ratio. For example, the variation may be detected by evaluating dispersion of the values of the oxygen saturations at measurement timings. For example, the evaluation of dispersion is performed by evaluating the magnitude of the secondary difference of oxygen saturations. The secondary difference of oxygen saturations is calculated in the following manner. The oxygen saturation which is calculated at the n-th measurement timing is indicated by S(n). The primary difference ΔS(n) of oxygen saturations is calculated by ΔS(n) = S(n) - S(n - 1). The secondary difference Δ²S(n) of oxygen saturations is calculated as the difference of primary differences ΔS(n), by Δ²S(n) = ΔS(n) - ΔS(n - 1) = {S(n) - S(n - 1)} - {S(n - 1) - S(n-2)} = S(n) - 2 × S(n - 1) + S(n - 2). The detecting section 112 may evaluate dispersion of the values of the oxygen saturations by, for example, comparing the sum of absolute values of the secondary differences Δ²S(n) at measurement timings with a predetermined threshold. As an index for evaluating dispersion of the values of oxygen saturations, the detecting section 112 may calculate the dispersion, standard deviation, or the like of the values of the oxygen saturations. In the case where the absolute value of the primary difference ΔS(n) of oxygen saturations at a certain measurement timing is larger than a predetermined threshold, the detecting section 112 may detect variation. Alternatively, the detecting section 112 may detect variation by evaluating the sum of absolute values of the primary differences ΔS(n) of oxygen saturations at measurement timings. Alternatively, the detecting section 112 may detect variation in multiple levels by using values, various indexes, or the like indicating the degree of variation, or detect variation in a binary manner such as "Variation exist" or "No variation."

The evaluating section 113 evaluates influences of body motion noises, external light noises, and the like contained in the pulse waveform acquired by the acquiring section 111, based on the variation of the oxygen saturation detected by the detecting section 112, and evaluates the reliability of the pulse waveform. The evaluating section 113 evaluates that the larger the variation of the oxygen saturation detected by the detecting section 112, the larger the influences of noises contained in the pulse waveform. Namely, it is evaluated that the reliability of the pulse waveform is low. Conversely, it is evaluated that the smaller the variation of the oxygen saturation, the smaller the influences of noises contained in the pulse waveform. Namely, it is evaluated that the reliability of the pulse waveform is high. The relationships between variation of the oxygen saturation and the reliability of the pulse waveform will be described in detail later.

The evaluating section 113 further evaluates the reliability of the physiological parameter (analysis item of the pulse wave analysis) which is acquired by performing the pulse wave analysis on the pulse waveform, in accordance with the reliability of the pulse waveform. This is realized by, for example, predetermining relationships between the reliability of the pulse waveform and the reliabilities of physiological parameters by experiments, simulation, or experience, and then previously storing table information indicating the relationships in the storage device 140.

The analyzing section 114 performs various pulse wave analyses on the pulse waveform detected by the detecting section 112, to acquire various physiological parameters. Examples of the physiological parameters acquired by the pulse wave analyses are the pulse wave amplitude, respiratory variation of the pulse wave amplitude, the pulse interval and the regularity of the pulse interval, the dicrotic wave, the rising angle of the pulse wave, the HRV (Heart Rate Variability), the respiratory rate, and the like. For example, the base line of the pulse wave is synchronized with the respiration, and therefore the respiratory rate may be acquired by analyzing variation of the base line from the pulse waveform.

The determining section 115 determines the condition of the subject based on the various physiological parameters acquired by the analyzing section 114. The condition of the subject determined by the determining section 115 includes the heart beat condition such as arrhythmia, various shock conditions such as blood flow distributive shock and hypovolemic shock, and the condition of drug administration. For example, the determining section 115 may determine whether arrhythmia has occurred or not, based on the regularity of the pulse interval. The determining section 115 may further determine the degree of expansion/contraction of peripheral vessels, and condition of the systemic blood flow in the living body, the blood pressure, and the like based on the pulse wave amplitude. The determining section 115 may further determine the volume and the like of blood circulating in the living body, based on the respiratory variation of the pulse wave amplitude. The determining section 115 may further determine the systemic vascular resistance and the like based on the condition of the dicrotic wave. The determining section 115 may further determine the heart contractility and the like based on the rising angle of the pulse wave. The determining section 115 may further determine the condition of autonomic nerves based on the HRV.

The outputting section 116 outputs physiological parameters acquired by the analyzing section 114, to the displaying section 150 and the like. The outputting section 116 may select a physiological parameter(s) which is to be output, based on a result of the evaluation of the pulse waveform by the evaluating section 113. For example, the outputting section 116 outputs a physiological parameter(s) which is determined by the evaluating section 113 to be high in reliability, to the displaying section 150.

### (Relationships between variation of oxygen saturation and reliability of pulse waveform)

Next, the relationships between variation of the oxygen saturation and the reliability of the pulse waveform will be described.

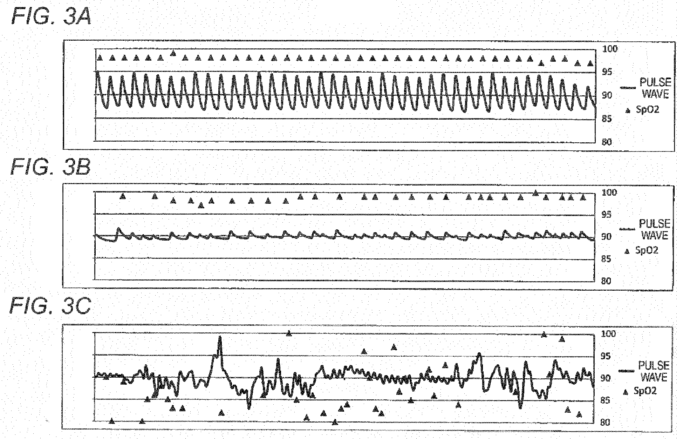

Figs. 3A to 3C illustrate examples of results of measurements of the pulse waveform and the oxygen saturation.

Fig. 3A illustrates results of measurements of the pulse waveform and the oxygen saturation which were performed on a subject having a normal pulse, Fig. 3B illustrates results of measurements of the pulse waveform and the oxygen saturation which were performed on a subject having an arrhythmia, and Fig. 3C illustrates results of measurements of the pulse waveform and the oxygen saturation which were performed on a subject in whom body motion noises occurred. In Figs. 3A to 3C, the curve graphs indicate the pulse wave, and the triangular symbols indicate the value of the oxygen saturation.

In the case of a a subject having a normal pulse, as illustrated in Fig. 3A, the results of measurements of the oxygen saturation show small variation and are stable. Also in the case of a subject having an arrhythmia, as illustrated in Fig. 3B, the results of measurements of the oxygen saturation show small variation and are stable in a similar manner as the case of the normal subject, although the pulse waveform and the pulse interval largely vary and are unstable in each pulse.

By contrast, in the case where body motion noises occurred, as illustrated in Fig. 3C, the results of measurements of the oxygen saturation largely vary and are unstable. The arterial oxygen saturation gradually changes, and its value does not sharply increase or decrease, nor unstably change. In the case where the oxygen saturation largely varies and is unstable, therefore, it is possible to determine that body motion noises, external light noises, and the like are contained in the pulse waveform, and the reliability of the pulse waveform is low.

### (Summary of process in medical apparatus 100)

Next, the procedure of processes which are executed in the medical apparatus 100 will be described.

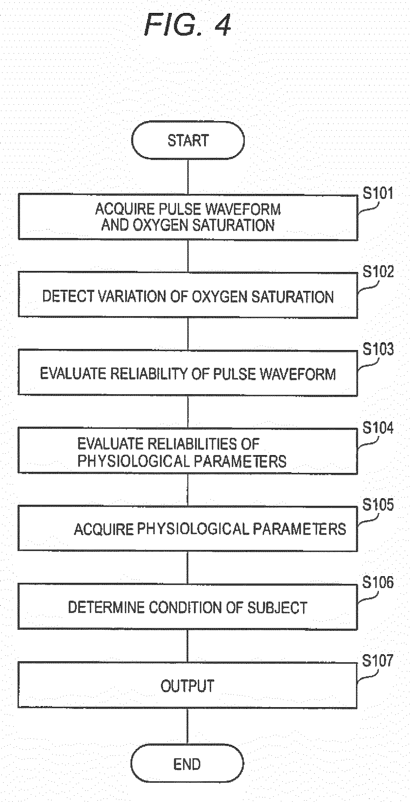

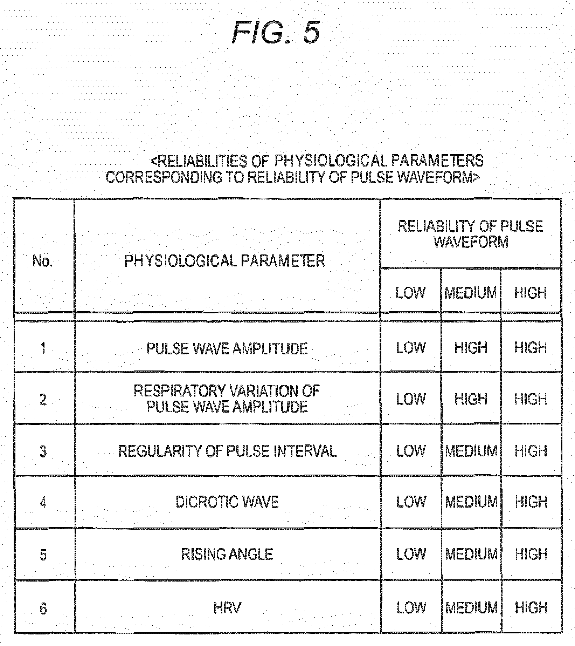

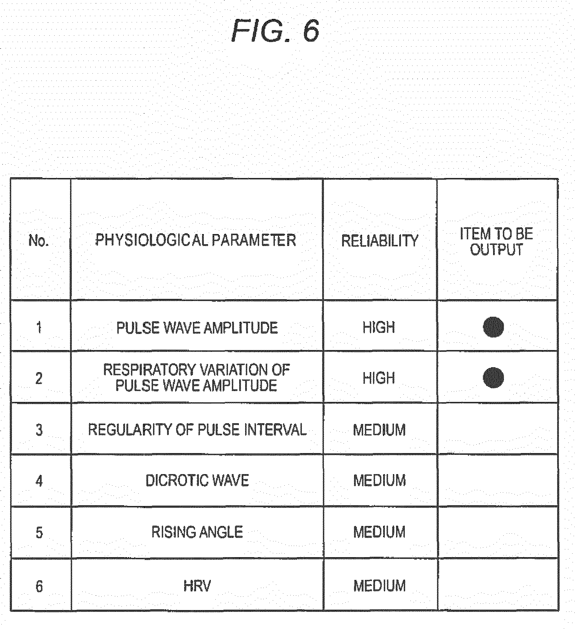

Fig. 4 is a flowchart illustrating the procedure of a a physiological parameter analyzing process executed in the medical apparatus, Fig. 5 illustrates an example of table information indicating relationships between the reliability of the pulse waveform and the reliabilities of physiological parameters, and Fig. 6 illustrates a process of selecting a physiological parameter which is to be output, based on the reliability of the physiological parameter. The processes of the medical apparatus 100 illustrated in the flowchart of Fig. 4 are stored as programs in the storage device 140 of the medical apparatus 100, and executed when the CPU 110 controls the sections.

Firstly, the medical apparatus 100 acquires the pulse waveform and the oxygen saturation from the subject (step S101). Specifcally, the CPU 110 of the medical apparatus 100 calculates the absorbances of the arterial blood based on the light beams of two wavelengths detected by the light detector 180, and acquires the arterial pulse waveform based on the calculated absorbances. The CPU 110 calculates and acquires the oxygen saturation of the arterial blood from a ratio of the absorbances with respect to the light beams of two wavelengths. The CPU 110 continuously acquires the pulse waveform and the oxygen saturation. The CPU 110 stores the acquired pulse waveform and oxygen saturation in the storage device 140.

Then, the medical apparatus 100 detects variation of the oxygen saturation (step S102). Specifically, the CPU 110 of the medical apparatus 100 evaluates dispersion of the values of oxygen saturations which are continuously acquired in step S101, thereby detecting variation of the oxygen saturation. For example, the evaluation of dispersion is performed by calculating and evaluating the secondary difference of the oxygen saturations. The CPU 110 determines that the larger the dispersion of the values of oxygen saturations, the larger the variation of the oxygen saturation, and also that the smaller the dispersion of the values of oxygen saturations, the smaller the variation of the oxygen saturation. The CPU 110 stores the detection result of the variation of the oxygen saturation in the storage device 140.

Then, the medical apparatus 100 evaluates the reliability of the pulse waveform (step S103). Specifically, the CPU 110 of the medical apparatus 100 evaluates influences of noises such as body motion noises and external light noises contained in the pulse waveform, based on the variation of the oxygen saturation detected in step S102, and evaluates the reliability of the pulse waveform. The CPU 110 evaluates that the larger the variation of the oxygen saturation, the larger the influences of noises contained in the pulse waveform, and the lower the reliability of the pulse waveform. Conversely, the CPU 110 evaluates that the smaller the variation of the oxygen saturation, the smaller the influences of noises contained in the pulse waveform, and the higher the reliability of the pulse waveform. The CPU 110 stores the evaluation result of the reliability of the pulse waveform in the storage device 140.

Then, the medical apparatus 100 evaluates the reliabilities of physiological parameters (step S104). Specifically, the CPU 110 of the medical apparatus 100 evaluates the reliabilities of the physiological parameters which are acquired by performing the pulse wave analysis on the pulse waveform, in accordance with the reliability of the pulse waveform which is evaluated in step S103. In the embodiment, as illustrated in Fig. 5, the storage device 140 of the medical apparatus 100 stores the table information indicating relationships between the reliability of the pulse waveform and the reliabilities of the physiological parameters. In the example of Fig. 5, in the case where the reliability of the pulse waveform is "Low," the reliabilities of all the physiological parameters are evaluated as "Low." In the case where the reliability of the pulse waveform is "Medium," the reliabilities of the pulse wave amplitude and the respiratory variation of the pulse wave amplitude are evaluated as "High," and the reliabilities of the other physiological parameters are evaluated as "Medium." In the case where the reliability of the pulse waveform is "High," the reliabilities of all the physiological parameters are evaluated as "High."

Then, the medical apparatus 100 analyzes the pulse waveform to acquire physiological parameters (step S105). Specifically, the CPU 110 of the medical apparatus 100 analyzes the pulse waveform acquired in step S101, to acquire physiological parameters such as the pulse wave amplitude, respiratory variation of the pulse wave amplitude, the pulse interval and the regularity of the pulse interval, the dicrotic wave, the rising angle of the pulse wave, the HRV (Heart Rate Variability), and the respiratory rate.

Then, the medical apparatus 100 determines the condition of the subject based on the physiological parameters (step S106). Specifically, the CPU 110 of the medical apparatus 100 determines the condition of the subject based on the various physiological parameters which are acquired in step S105. For example, the CPU 110 determines whether arrhythmia has occurred or not, based on the regularity of the pulse interval. The CPU 110 further determines the degree of expansion/contraction of peripheral vessels, and condition of the systemic blood flow in the living body, the blood pressure, and the like based on the pulse wave amplitude. The CPU 110 further determines the volume and the like of blood circulating in the living body, based on the respiratory variation of the pulse wave amplitude. The CPU 110 further determines the systemic vascular resistance and the like based on the condition of the dicrotic wave. The CPU 110 further determines the heart contractility and the like based on the rising angle of the pulse wave. The CPU 110 further determines the condition of autonomic nerves based on the HRV.

Then, the medical apparatus 100 outputs, together with the pulse waveform and the oxygen saturation, the physiological parameters and the condition of the subject which is determined based on the physiological parameters (step S107). In the embodiment, as illustrated in Fig. 6, the medical apparatus 100 selects a physiological parameter(s) which is to be output, based on the reliabilities of the physiological parameters which are evaluated in step S104. In the example of Fig. 6, "Pulse wave amplitude" the reliabilities of which is determined to be high and "Respiratory variation of pulse wave amplitude" the reliabilities of which is determined to be high are selected as the items to be output. The CPU 110 outputs the physiological parameters which are selected as the items to be output, to the displaying section 150. The CPU 110 further outputs the condition of the subject which is determined by using these physiological parameters in step S105, to the displaying section 150.

According to the medical apparatus 100 of the embodiment, as described above, the variation of the oxygen saturation is detected, and the pulse waveform which is acquired for the purpose of analysis of a pulse wave is evaluated based on the detected variation of the oxygen saturation. When the variation of the oxygen saturation is detected, it is possible to evaluate noise mixture in the pulse wave. Therefore, the medical apparatus 100 may output the physiological parameters with consideration of noise mixture. Consequently, the user may adequately determine the condition of the subject based on the physiological parameters output from the medical apparatus 100.

Moreover, the medical apparatus 100 evaluates the reliability of the pulse waveform itself as evaluation of the pulse waveform. As a result, the reliability of the pulse waveform itself is evaluated, and therefore the subsequent processes of analyzing the pulse wave, determining the condition of the subject, and selecting the item to be output may be adequately performed in accordance with the reliability of the pulse waveform.

Moreover, the medical apparatus 100 determines that the larger the variation of the values of oxygen saturations, the smaller the reliabilities of the pulse waveform and the physiological parameters. Therefore, the reliabilities of the pulse waveform and the physiological parameters may be adequately determined with consideration of influences of body motion noises, external light noises, and the like.

Moreover, the medical apparatus 100 performs the pulse wave analysis on the pulse waveform to acquire the pulse interval, and determines whether arrhythmia has occurred or not, based on the acquired pulse interval. Therefore, it is possible to correctly sense arrhythmia while eliminating influences of body motion noises, external light noises, and the like. Also in the case where the pulse interval is unstable, and arrhythmia is suspected, when the variation of the oxygen saturation is large, for example, it may be determined that arrhythmia has not occurred, and there is a possibility that the phenomenon is affected by noises. By contrast, in the case where the pulse interval is unstable, and the variation of the oxygen saturation is small, it may be determined that noises exert less influence, and there is a high possibility that arrhythmia has occurred.

Moreover, the medical apparatus 100 selects a physiological parameter(s) of the pulse wave analysis which is to be output, in accordance with the reliability. According to the configuration, it is possible to output only a physiological parameter(s) in which the reliability is high. Therefore, a situation where the user erroneously determines the condition of the subject based on a physiological parameter(s) having a low reliability may be suppressed from occurring.

The present invention is not limited to the above-described embodiment, and may be variously modified within the scope of the appended claims.

For example, the embodiment in which, after the reliability of the pulse waveform is evaluated in step S103 of Fig. 4, the reliability of each physiological parameter item is evaluated in step S104 has been described. However, the method for evaluating the pulse waveform is not limited to this. For example, the medical apparatus 100 may not evaluate the reliability of the pulse waveform itself, and may directly evaluate the reliability(ies) of a physiological parameter(s) based on variation of the oxygen saturation. This is realized by predetermining relationships between variation of the oxygen saturation and the reliabilities of physiological parameters by experiments, simulation, or experience, and then previously storing table information indicating the relationships in the storage device 140. When the reliabilities of physiological parameters are directly evaluated based on variation of the oxygen saturation in this way, the process may be simplified.

Although, in the embodiment, the example in which, in step S105 of Fig. 4, the pulse wave analysis is performed on all acquirable physiological parameters, and all the physiological parameters are acquired has been described, the present invention is not limited to this. For example, the medical apparatus 100 may perform the pulse wave analysis on physiological parameters which are evaluated to have a high reliability in step S104, and acquire only physiological parameters having a high reliability. Alternatively, the medical apparatus 100 may perform the pulse wave analysis on a physiological parameter(s) which is evaluated to have a reliability higher than a predetermined reference level. According to the alternative, a waste analyzing process of acquiring a physiological parameter in which the reliability is low, and which is not useful may be omitted, and the speed and efficiency of the whole process may be improved.

Although, in the embodiment, the example in which, in step S107 of Fig. 4, only a physiological parameter(s) having high reliability is selected as the item to be output has been described, the method for outputting a physiological parameter(s) is not limited to this. For example, all physiological parameters may be output together with information indicating the reliabilities of the physiological parameters. According to the configuration, a larger number of sets of information indicating the condition of the subject may be output together with the reliabilities of the information, and therefore the condition of the subject may be determined more adequately. Alternatively, a physiological parameter(s) which is evaluated to have a reliability higher than a predetermined reference level may be output as an item(s) to be output. According to the configuration, for example, physiological parameters in which a certain degree of reliability is ensured may be output while excluding physiological parameters in which the reliability is very low. As described above, the outputting conditions of a physiological parameter may be flexibly set in accordance with various use conditions such as the condition of the subject and requests of the user, and therefore the usability of the user may be improved, and the condition of the subject may be determined more adequately.

The means and methods for performing the various processes in the medical apparatus of the above-described embodiments may be realized by either of a dedicated hardware circuit and a programmed computer. For example, the programs may be provided by a computer readable recording medium such as a flexible disk or a CD-ROM, or provided online through a network such as the Internet. In this case, the programs which are recorded on a computer readable recording medium are usually transferred and stored in a storage section such as a hard disk drive. Alternatively, the programs may be provided in the form of standalone application software, or incorporated as one function of the medical apparatus into software for the apparatus.

## Claims

1. A medical apparatus comprising:
a light emitter that is configured to emit light beams of different wavelengths to living tissue;
a light detector that is configured to detect the light beams of different wavelengths emitted from the light emitter, through the living tissue;
an acquiring section that calculates absorbances of arterial blood for the respective light beams of different wavelengths, based on the light beams detected by the light detector, that acquires a pulse waveform based on the absorbances, and that acquires a parameter corresponding to an oxygen saturation from a ratio of the absorbances;
a detecting section that detects variation of the parameter corresponding to the oxygen saturation acquired by the acquiring section; and
an evaluating section that evaluates the pulse waveform that is acquired for a purpose of pulse wave analysis by the acquiring section, based on a result of the detection by the detecting section.

2. The medical apparatus according to claim 1, wherein the parameter corresponding to the oxygen saturation is an arterial oxygen saturation.

3. The medical apparatus according to claim 1, wherein the parameter corresponding to the oxygen saturation is the ratio of the absorbances.

4. The medical apparatus according to claim I, wherein the parameter corresponding to the oxygen saturation is a function of the ratio of the absorbances which corresponds one-to-one to an arterial oxygen saturation.

5. The medical apparatus according to claim 1, wherein the evaluation of the pulse waveform is preformed by evaluating reliability of the pulse waveform itself.

6. The medical apparatus according to claim 1, wherein the evaluation of the pulse waveform is preformed by evaluating reliability of a physiological parameter that is acquired by pulse wave analysis performed on the pulse waveform.

7. The medical apparatus according to any one of claims 1 to 6, wherein the evaluating section determines that the larger the variation, the lower the reliability.

8. The medical apparatus according to any one of claims 1 to 7 further comprising:
an analyzing section that performs pulse wave analysis on the pulse waveform to acquire a pulse interval as a physiological parameter; and
a determining section that determines whether arrhythmia has occurred or not, based on the pulse interval acquired by the analyzing section.

9. The medical apparatus according to any one of claims 1 to 8 further comprising an outputting section that outputs a physiological parameter that is acquired by performing pulse wave analysis on the pulse waveform.

10. The medical apparatus according to claim 9, wherein the outputting section selects a physiological parameter which is to be output, in accordance with the reliability.

11. The medical apparatus according to claim 9 or 10, wherein the outputting section outputs the physiological parameter together with the reliability.

12. A method for analyzing a physiological parameter, the method being used in a medical apparatus having a light emitter that is configured to emit light beams of different wavelengths to living tissue including arterial blood, and a light detector that is configured to detect the light beams of different wavelengths emitted from the light emitter, through the living tissue, the method comprising:
calculating absorbances of the arterial blood for the respective light beams of different wavelengths, based on the light beams detected by the light detector;
acquiring a pulse waveform based on the absorbances and a parameter corresponding to an oxygen saturation from a ratio of the absorbances;
detecting variation of the parameter corresponding to the oxygen saturation acquired in the acquiring; and
evaluating the pulse waveform which is acquired for a purpose of pulse wave analysis in the acquiring, based on a result of the detection in the detecting.

13. A program executed in an apparatus for processing a physiological parameter obtained from a light emitter that is configured to emit light beams of different wavelengths to living tissue and a light detector that is configured to detect the light beams of different wavelengths emitted from the light emitter, through the living tissue, wherein the program cause the apparatus to execute:
calculating absorbances of the arterial blood for the respective light beams of different wavelengths, based on the light beams detected by the light detector;
acquiring a pulse waveform based on the absorbances and a parameter corresponding to an oxygen saturation from a ratio of the absorbances;
detecting variation of the parameter corresponding to the oxygen saturation acquired in the acquiring; and
evaluating the pulse waveform which is acquired for a purpose of pulse wave analysis in the acquiring, based on a result of the detection in the detecting.
